# EUROPÄISCHE PATENTSCHRIFT

(11) **EP 4 080 195 B1**
(45) Veröffentlichungstag und Bekanntmachung des Hinweises auf die Patenterteilung: **07.06.2023**
(21) Anmeldenummer: 21169141.5
(22) Anmeldetag: 19.04.2021
(51) Int. Cl.: G01N 21/3563, G01N 21/359, G01N 21/27, G01N 21/84, G01N 33/00

(54) **VERFAHREN ZUR BESTIMMUNG DER KLEBSTOFF-PENETRATION IN HOLZ MITTELS NAH-INFRAROT-SPEKTROSKOPIE**
METHOD OF DETERMINING ADHESIVE PENETRATION IN WOOD USING NEAR INFRARED SPECTROSCOPY
PROCÉDÉ DE DÉTERMINATION DE LA PÉNÉTRATION D'ADHÉSIF DANS BOIS UTILISANT LA SPECTROSCOPIE INFRAROUGE PROCHE

(43) Veröffentlichungstag der Anmeldung: 26.10.2022
(73) Patentinhaber: Flooring Technologies Ltd., Kalkara SCM1001 (MT)
(72) Erfinder: Kalwa, Norbert, Dr., 32805 Horn-Bad Meinberg (DE)
(74) Vertreter: Maikowski & Ninnemann Patentanwälte Partnerschaft mbB

(56) Entgegenhaltungen:
- EP-A1- 3 327 424
- WO-A1-2007/021235
- CN-A- 105 334 179

## Beschreibung

Die vorliegende Erfindung betrifft ein Verfahren zur Bestimmung der Klebstoffpenetration in mindestens ein poröses Beschichtungsmaterial, welche mit mindestens einer Trägerplatte und mindestens einer auf der Trägerplatte angeordneten Klebstoffschicht verpresst ist, wobei während des Pressvorganges der Klebstoff in das mindestens eine poröse Beschichtungsmaterial penetriert bzw. aufsteigt.

### Beschreibung

Bodenbeläge werden zunehmend mit verschiedenen Beschichtungsmaterialien wie Leder, Filz oder Echtholz versehen, um gestiegene Kundenwünsche zu erfüllen.

Bei der Herstellung von Bodenbelägen mit einer Echtholzoberfläche werden verschiedene Technologien eingesetzt. Ein Ansatz besteht darin relativ dicke Echtholzfurniere bis zu einer Stärke von mehreren Millimetern auf quer dazu angeordnete Holzlagen aufzuleimen. Als Unterlage werden quer zu der Mittellage liegenden Holzschichten eingesetzt.

Ein anderer Ansatz besteht darin, die Holzlagen durch Holzwerkstoffe zu ersetzen. Somit wird in hierbei ein Echtholzfurnier auf einen Holzwerkstoffträger (HDF, Spanplatte, OSB usw.) aufgeleimt. Die hierbei verwendeten Furniere weisen typischerweise eine geringere Dicke auf, was eine geringere mechanische Festigkeit bedingt, als relativ hohe Furnierstärken.

Der Vorteil der Verwendung von dünnen Echtholzfurnieren besteht in den günstigeren Produktions- und Materialkosten. Allerdings erfordert die Verwendung von Furnieren eine geeignete Oberflächenvergütung. Eine mögliche Oberflächenvergütung besteht dabei üblicherweise aus einer Lackierung auf Basis von UV- oder ESH-Lacken.

Zur Verklebung der Furniere auf dem Träger werden meist Harnstoff-, PMDI- oder PVAc-Leime mit Härter eingesetzt. Auf der Rückseite des Produktes befindet sich meist ein Gegenzug auf Basis von Furnier, der die Spannungssymmetrie im Produkt gewährleisten soll. Zudem wird dadurch auch der Aspekt eines Holzfußbodens zusätzlich unterstützt.

Ein Problem stellt bei diesem Produkt die nur eingeschränkte Möglichkeit dar, das Produkt bei Beschädigung zu reparieren. Dies ist umso gravierender, weil die mechanische Stabilität der Furnierlage wegen der geringen Rohdichte des Furniers (300 - 500 kg/m³) nicht besonders hoch ist. Bei mechanischer Beschädigung durch z. B. herabfallende Gegenstände werden bei einem derartigen Produkt also schnell tiefe Eindrücke erzeugt.

Eine Lösung für dieses Problem liefert die WO 2015/105456 A1, bei der ein Gemisch von Holzmehl und Melaminharzpulver auf eine Holzwerkstoffplatte aufgestreut wird und anschließend zusammen mit einem Furnier auf die Holzwerkstoffplatte aufgepresst wird. Hier wird ein möglichst weitgehendes Penetrieren des Melaminharzes gewünscht, eine direkte Kontrolle der Penetrationshöhe des Harzes in das Furnier ist allerdings nicht möglich.

Die oben bezüglich der verleimten Furniere geschilderten Probleme werden ebenfalls durch eine neue Technologie im Wesentlichen behoben. Dabei wird das Furnier in einer Kurztaktpresse mit Hilfe eines mit Melaminharz imprägnierten Papiers (z. B. ein Overlay ) auf den Holzwerkstoffträger aufgepresst. Die Pressparameter liegen dabei bei ca. T > 150°C, p > 30 × 10⁵ Pa (30 bar) und t > 30 sec. Mit dieser Technologie lassen sich ebenfalls Furnierfussböden mit Furnieren herstellen, die eine Stärke von ca. 0,5 mm besitzen. Von entscheidender Bedeutung ist dabei, dass auch hier beim Presssvorgang das Melaminharz möglichst weit in das Furnier aufsteigt. Dadurch erfolgt zum einen eine Armierung des Furniers mit dem Kunstharz und zum anderen wird das durch die Verpressung komprimierte Furnier in dem Zustand fixiert. Allerdings sollte das Melaminharz nicht aus dem Furnier austreten, da dies zu Verfärbungen in der Oberfläche und zu Haftungsproblemen beim anschließenden Lackieren oder Ölen führt. Ein Problem ist nun, dass die Bestimmung der Güte der Armierung (d.h. das Aufsteigen des Melaminharzes in das Furnier) nicht zerstörungsfrei bzw. on-line durchgeführt werden kann. Dies ist umso gravierender, da je nach Kollektion bzw. Nutzungsklasse sowohl unterschiedliche Holzfurniere als auch unterschiedliche Furnierstärken verarbeitet werden. Zudem können sich auch Furniere gleicher Holzart aus unterschiedlichen Regionen bezüglich ihrer Eigenschaften unterscheiden.

Es ergeben sich somit folgende Nachteile: keine zerstörungsfreie Prüfung des Prozesses möglich; höhere Kosten durch Qualitätsbestimmung und Nachjustierungen an Pressparametern notwendig.

CN 105334179 A beschreibt ein Verfahren zur Bestimmung der Eindringtiefe von Holzkleber durch Fourier-transformierte Infrarot(FTIR) - Mikrospektroskopie. Eine mittels Holzkleber verklebte Holzprobe wird von einem FTIR-Mikrospektroskop gescannt. Für einen ausgewählten Bildausschnitt wird ein Bild erstellt, wobei nur die Signale des Holzklebers verwendet werden. Aus den Hell-Dunkel-Kontrasten dieses FTIR-Bildes wird dann die Eindringtiefe ermittelt.

Der Erfindung liegt die technische Aufgabe zu Grunde eine alternative zerstörungsfreie Methode zu entwickeln, die eine Bestimmung des Grades der Klebstoffpenetration in poröse Beschichtungsmaterialien, wie Furniere, Vliese, Leder u.a. ermöglicht. Die Methode sollte möglichst schnell Ergebnisse liefern, damit in der Produktion möglichst geringe oder gar keine Stillstandzeiten wegen der Qualitätsbestimmung entstehen. Die Klebstoffpenetration soll bereits unmittelbar hinter der Presse möglich sein und eine kontinuierliche Überwachung dieses Parameters ermöglichen.

Diese Aufgabe wird durch ein Verfahren mit den Merkmalen des Anspruchs 1 gelöst.

Demnach wird ein Verfahren zur Bestimmung der Klebstoffpenetration bzw. Penetrationshöhe des Klebstoffes in mindestens ein poröses Beschichtungsmaterial bereitgestellt, wobei das mindestens eine poröse Beschichtungsmaterial mit mindestens einer Trägerplatte und mindestens einer auf der Trägerplatte angeordneten Klebstoffschicht verpresst ist, und wobei während des Pressvorganges der Klebstoff in das mindestens eine poröse Beschichtungsmaterial penetriert bzw. aufsteigt. Das vorliegende Verfahren umfasst die folgenden Schritte:
- Aufnahme von mindestens einem NIR-Spektrum von mehreren Referenzproben mit jeweils unterschiedlichem Werten für die Klebstoffpenetration in ein poröses Beschichtungsmaterial unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1000 nm und 1300 nm, noch vorteilhafter zwischen 1100 nm und 1250 nm und/oder zwischen 1400 nm und 1550 nm;
- Bestimmung der Klebstoffpenetration in das poröse Beschichtungsmaterial der genannten Referenzproben mittels eines mechanischen Abtrages der porösen Materialoberfläche;
- Zuordnung der mittels mechanischen Abtrages bestimmten Klebstoffpenetration zu den aufgenommenen NIR-Spektren der genannten Referenzproben; und
- Erstellung eines Kalibriermodells für den Zusammenhang zwischen den spektralen Daten der NIR-Spektren und den dazugehörigen Klebstoffpenetrationen der Referenzproben mittels einer multivariaten Datenanalyse;
- Verpressen von mindestens einem porösem Beschichtungsmaterial mit mindestens einer Trägerplatte und mindestens einer auf der Trägerplatte angeordneten Klebstoffschicht,
- Aufnehmen von mindestens einem NIR-Spektrum der mit der Trägerplatte und der Klebstoffschicht verpressten porösem Beschichtungsmaterial unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1000 nm und 1300 nm, noch vorteilhafter zwischen 1100 nm und 1250 nm und/oder zwischen 1400 nm und 1550 nm; und
- Bestimmen der Klebstoffpenetration in das mindestens eine poröse Beschichtungsmaterial durch Vergleich des für das poröse Beschichtungsmaterial aufgenommenen NIR-Spektrums mit dem erstellten Kalibriermodell.

Gemäß dem vorliegenden Verfahren wird ein NIR-Spektrum der porösen Materialoberfläche aufgenommen. Es wird eine NIR-Strahlung erzeugt und auf die zu analysierende Trägermaterial-Probe mit der Materialoberfläche geleitet, wo die NIR-Strahlung mit den Bestandteilen der Probe wechselwirkt und reflektiert bzw. gestreut wird. Ein NIR-Detektorfängt die reflektierte bzw. gestreute NIR-Strahlung auf und erzeugt ein NIR-Spektrum, das die gewünschten chemischen Informationen der Probe beinhaltet. Bei dieser Messung wird in einer Sekunde eine Vielzahl von einzelnen NIR-Messungen durchgeführt, sodass auch eine statistische Absicherung der gemessenen Werte gewährleistet wird. Die NIR-Spektroskopie zusammen mit der (unten angeführten) Multivariaten Datenanalyse bietet eine Möglichkeit an, einen direkten Bezug zwischen den spektralen Informationen (NIR-Spektren) und den zu bestimmenden Parametern des aufgebrachten porösen Beschichtungsmaterials, wie z.B. einer Furnierlage herzustellen.

Das vorliegende Verfahren nutzt den Umstand aus, dass die NIR-Strahlung zwar durch die Beschichtungsmaterialien jedoch nicht durch die Trägerplatte hindurch dringt, sondern an der Oberfläche der Trägerplatte reflektiert bzw. gestreut wird. Die reflektierte bzw. gestreute NIR-Strahlung wird von dem NIR-Detektor erfasst, und das ermittelte NIR-Spektrum wird zur Bestimmung der gewünschten Parameter (hier Penetrationshöhe des Klebstoffes in das Beschichtungsmaterial) verwendet.

Das aufgenommene NIR-Spektrum ermöglich es in Kombination mit einer Prüfung des Prozentsatzes der Penetration über ein mechanisches Abtragen der porösen Materialoberfläche eine Korrelation zu erzeugen. Es hat sich nämlich überraschenderweise gezeigt, dass je nachdem wie weit der Klebstoff z.B. Polyurethan-Klebstoff in das poröse Beschichtungsmaterial aufsteigt, eine Signalerhöhung für den Klebstoffpeak zu beobachten ist.

Es werden zunächst Referenzproben des einer mit einem porösen Beschichtungsmaterial und Klebstoffschicht verpressten Trägerplatte bereitgestellt. Wesentlich ist, dass die Referenzprobe gleichartig zu der zu vermessenden Probe ist; d.h. insbesondere die Klebstoffschicht und poröses Beschichtungsmaterial der Referenzprobe weisen die gleiche Zusammensetzung wie die zu vermessende Klebstoffschicht und poröses Beschichtungsmaterial auf. Die Gleichartigkeit von zu vermessender Probe und Referenzprobe ist insbesondere bei Verwendung von Klebstoffschichten mit Zusatzstoffen wie Flammschutzmitteln, Fasern, weiteren Additiven wesentlich.

Von diesen Referenzproben werden zumindest ein NIR-Spektrum in einem Wellenlängenbereich zwischen 500 nm und 2500 nm, bevorzugt zwischen 700 nm und 2000 nm, insbesondere bevorzugt zwischen 900 nm und 1700 nm aufgenommen.

Diese Referenzproben werden ebenfalls einer nicht-spektroskopischen Analyse zur Bestimmung der gewünschten Parameter, d.h. im vorliegenden Fall eines mechanischen Abtrages der porösen Materialoberfläche, zugeführt.

Aus den jeweils mittels der nicht-spektroskopischen Analyse ermittelten Parameter für die Referenzproben wird ein Mittelwert gebildet, welcher dann den jeweils aufgenommen NIR-Spektren dieser Referenzproben zugeordnet wird, und es wird ein Kalibriermodell für den Zusammenhang zwischen den spektralen Daten der NIR-Spektren der Referenzproben und den dazugehörigen Parameterwerten mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens ein poröses Beschichtungsmaterial mit Klebstoffschicht und Trägerplatte verpresst und mindestens ein NIR-Spektrum des verpressten poröses Beschichtungsmaterials aufgenommen. Der gewünschte Parameter des porösen Beschichtungsmaterials (hier die Klebstoffpenetration bzw. Penetrationshöhe in das poröse Beschichtungsmaterial) kann dann durch Vergleich des für das verpresste poröses Beschichtungsmaterial aufgenommenen NIR-Spektrums mit dem erstellten Kalibriermodell bestimmt werden.

Es ist somit möglich, aus einem einzigen für die zu vermessende Probe ermittelten NIR-Spektrum durch einen automatisierten Vergleich bzw. Abgleich mit den für die jeweiligen Parameter erstellten Kalibriermodellen gleichzeitig mehrere interessierende Parameter des mit der Trägerplatte verpressten porösen Beschichtungsmaterial zu bestimmen.

Ein Vergleich und die Interpretation der NIR-Spektren erfolgt sinnvollerweise über den gesamten aufgenommenen Spektralbereich. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

In einer weiteren Ausführungsform ist vorgesehen, für die Erstellung des Kalibriermodells spektrale Daten aus dem NIR- Spektralbereich zwischen 1000 nm und 1300 nm, bevorzugt zwischen 1100 und 1250 nm und/oder zwischen 1400 nm und 1550 nm zu verwenden, die mittels geeigneter mathematischer Methoden vorbehandelt werden und anschließend der multivariaten Datenanalyse zugeführt werden.

Die Bedeutung einer Wellenlänge für die Vorhersage von Parametern des verpressten porösen Beschichtungsmaterials, wie z.B. der Klebstoffpenetration, aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell für die Bestimmung der Klebstoffmenge, dass der Wellenlängenbereich zwischen 1000 nm und 1300 nm und/oder zwischen 1400 nm und 1550 nm für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen (wie Transparenz der Schicht, Oberflächenbeschaffenheit der Klebstoffschicht oder des Trägermaterials usw.) zu berücksichtigen bzw. zu minimieren.

Zur Eliminierung von störenden Einflüssen (wie z.B. Beschaffenheit der Oberfläche des Trägermaterials oder des porösen Beschichtungsmaterials, Farbigkeit der Proben, Lichtstreuung an Feststoffpartikeln oder anderen Additiven usw.) ist es notwendig, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten. Dabei werden die Basislinieneffekte, die hauptsächlich durch die unterschiedliche Farbe der Proben verursacht werden, aus den Spektren entfernt, überlagernde Banden voneinander getrennt und die Abhängigkeit der Lichtstreuung an der Substratoberfläche berücksichtigt. So erfolgt die Datenvorbehandlung bevorzugt zur Reduzierung der Lichtstreuung an der rauen Oberfläche des Substrates. Bei der Messung liegt der Schwerpunkt der Kalibrierung und Datenvorbehandlung auf der Entfernung der Basislinienverschiebung.

Aus den vorbehandelten Daten wird mit Hilfe der multivariaten Datenanalyse ein Kalibriermodell entwickelt, das alle bei der Kalibrierung verwendeten Dekore beinhaltet.

Entsprechend erfolgen der Vergleich und die Interpretation der NIR-Spektren bevorzugt im Spektralbereich zwischen 1000 nm und 1300 nm und/oder zwischen 1400 nm und 1550 nm unter Verwendung der multivariaten Datenanalyse MDA. Bei multivariaten Analysemethoden werden in an sich bekannter Weise typischerweise mehrere statistische Variablen zugleich untersucht. Hierzu wird die in einem Datensatz enthaltene Zahl von Variablen reduziert, ohne gleichzeitig die darin enthaltene Information zu mindern.

Im vorliegenden Verfahren wurden für die Erstellung der Korrelation Reihenverpressungen durchgeführt, bei denen eine HDF (Faserplatte mit erhöhter Rohdichte) mit unterschiedlichen Mengen flüssigem und anschließend getrocknetem Klebstoff (wie PMDI und Polyvinylacetat-Leim) bestrichen wurde. Dann wurden auf diese HDF ein poröses Beschichtungsmaterial, z.B. ein Eichenfurnier mit einer Stärke von 0,5 mm, aufgelegt und verpresst. Von diesen Proben wurden NIR-Spektren angefertigt. Dabei zeigte sich, dass der Klebstoffpeak, je nach Penetration mehr oder weniger deutlich ausgeprägt war. Durch einen mechanischen Abtrag des porösen Materials wurde dann die Penetrationshöhe des Klebstoffes in das poröse Material bestimmt und mit den NIR-Spektren in Beziehung gesetzt. Dabei kann zur besseren Sichtbarmachung des Klebstoffes bzw. der Klebstofffront eine Einfärbung des Klebstoffs vorgenommen werden, die aber die NIR-Spektroskopie nicht stört.

Das vorliegende Verfahren ermöglicht die Bereitstellung der Messwerte in kurzer Zeit (online, bevorzugt ohne störende Zeitverzögerung) im Vergleich zu herkömmlichen (bekannten) Messverfahren. Die Messdaten können zur Qualitätssicherung, Forschung und Entwicklung, zur Prozesskontrolle, -regelung, -steuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Verbesserung der Anlagenverfügbarkeit.

In einer Ausführungsform des vorliegenden Verfahrens ist der mindestens eine Klebstoff ein Polyurethan-Klebstoff. Dabei wird ein Polyurethan-Klebstoff auf der Basis von aromatischen Polyisocyanaten, insbesondere Polydiphenylmethandiisocyanat (PMDI), Toluylendiisocyanat (TDI) und/oder Diphenylmethandiisocyanat (MDI) verwendet, wobei PMDI besonders bevorzugt ist. Das Isocyanat unterliegt bei seiner Verwendung als Bindemittel bzw. Klebstoff zwei chemischen Reaktionen. Zum einen bildet es in Gegenwart von Wasser Polyharnstoff aus. Parallel dazu erfolgt die Anbindung an die zu verklebenden Partikel bzw. Oberflächen durch die Reaktion der Isocyanate mit freien Hydroxy- oder Aminogruppen an der Oberfläche unter Ausbildung einer Urethan- oder Harnstoffbindung.

Die Menge des aufgetragenen Polyurethanklebstoffes, z.B. eines 100% PMDI Klebstoffsystems, beträgt 50 bis 150 g fl/m², bevorzugt 70 bis 120 g fl/m², insbesondere bevorzugt 90 bis 110 g fl/m², z.B. 100 g fl/m².

Bei Verwendung eines Polyurethanklebstoffes, wie PMDI-Klebstoff, sind im NIR-Spektrum charakteristische Peaks in Wellenlängenbereichen zwischen 1120 und 1250 nm mit Maxima bei 1130-1150 nm und 1200-1220 nm, und zwischen 1440 und 1540 nm zu erkennen.

In einer weiteren Ausführungsform wird ein Polyvinylester-Klebstoff, insbesondere ein Polyvinylacetat (PVAc)-Klebstoff, verwendet.

Die Menge des aufgetragenen Polyvinylester-Klebstoff z.B. eines PVAc- Klebstoffsystems mit 50 Gew% Feststoff, beträgt 100 bis 300 g fl/m², bevorzugt 150 bis 250 g fl/m², insbesondere bevorzugt 180 bis 220 g fl/m², z.B. 200 g fl/m².

Bei Verwendung eines Polyvinylester-Klebstoffes wie z.B. eines PVAc - Klebstoffs sind im NIR-Spektrum charakteristische Peaks in Wellenlängenbereichen zwischen 1160 und 1220 nm mit einem Maximum bei 1180-1200 nm, und zwischen 1420 und 1480 nm zu erkennen.

### Additiv

Wie bereits oben erwähnt, kann gemäß dem vorliegenden Verfahren auf oder in die mindestens eine Klebstoffschicht mindestens ein Additiv auf - oder eingebracht werden.

In einer bevorzugten Ausführungsform wird auf die (bevorzugt klebrige) Oberfläche der Klebstoffschicht mindestens ein Additiv aufgetragen. Das Additiv kann in flüssiger oder fester Form, insbesondere als partikelförmiger Feststoff (Staub, Pulver, Granulat), oder als Flüssigkeit oder Paste zum Beispiel mittels Sprühen, Spritzen, Gießen, Rakeln, Walzen, Streuen aufgebracht werden.

Es kann ein Additiv oder Mischungen von mehreren Additiven verwendet werden, wobei auch mehrere Additive nacheinander auftragbar sind.

Die zum Einsatz kommenden Additive können aus folgender Gruppe ausgewählt sein: Farbstoffe (Tinte), Pigmente (z.B. Farbpigmente, Metallpigmente oder reflektierende Pigmente) Flammschutzmittel (z.B. Ammoniumpolyphosphat, Tris(tri-bromneopentyl)phosphat, Zinkborat oder Borsäurekomplexe von mehrwertigen Alkoholen), Mittel zur Erhöhung der Leitfähigkeit, UV-Stabilisatoren, Bleichmittel, Hydrophobierungsmittel oder antimikrobielle Wirkstoffe.

Mögliche antimikrobielle Wirkstoffe können mindestens ein Biozid umfassen. Voraussetzung für die Auswahl eines geeigneten Biozids ist, das dieses der EU-Verordnung Nr. 528/2012 über das Inverkehrbringen von Biozid-Produkten entspricht. Biozide können entweder nach Produktarten wie Desinfektionsmittel und Schutzmittel oder nach deren Zielorganismen (Viruzide, Bakterizide, Fungizide etc.) eingeteilt werden. Vorliegend kann das mindestens eine Biozid ausgewählt sein aus einer Gruppe umfassend Benzalkoniumchlorid, Oktylammoniumchlorid, Chitosan, Phenylphenol, Kupfersulfat, Silbernitrat, Milchsäure, Nonansäure, Natriumbenzoat, 1-[[2-(2,4-dichlorophenyl)-4-propyl-1,3-dioxolan-2-yl]methyl]-1H-1,2,4-triazole, 2-octyl-2H-isothiazol-3-one, Thiazol-4-yl-1H-benzoimidazole, 3-lodo-2-propynylbutylcarbamat, Biphenyl-2-ol, Bronopol / Kalzium- Magnesiumoxid, Kupfer (II) oxid, 2-Pyridinthiol-1-oxid, Silberoxid, Silber-Kupfer-Zeolith. Die angeführten Wirkstoffe stammen aus Produktfamilien 2 und 9, die für antiviral wirkende Fußböden bereits genehmigt sind bzw. sich in Genehmigung befinden.

Bevorzugt ist das Additiv in dem auf der Oberfläche vorgesehenen Klebstoffes nicht lösbar bzw. nicht homogen lösbar. So wird gewährleistet, dass sich das Additiv nicht mit dem Klebstoff vermischt, sondern auf der Oberfläche verbleibt und dadurch mit dem porösen Beschichtungsmaterial in Kontakt kommen und in dieses penetrieren kann.

### Trägerplatte

In einer Ausführungsform des vorliegenden Verfahrens ist die mindestens eine Trägerplatte eine Platte aus einem Holzwerkstoff, insbesondere eine Span-, mitteldichte Faser (MDF)-, hochdichte Faser (HDF)-, Oriented Strand Board (OSB)- oder Sperrholzplatte, aus Kunststoff, einem Holzwerkstoff-Kunststoff-Gemisch oder einem Verbundwerkstoff, eine Zementfaserplatte, Gipsfaserplatte oder eine WPC-Platte (Wood Plastic Composites) oder eine SPC-Platte (Stone Plastic Composites).

Die Oberfläche des Trägermaterials kann oberflächenbehandelt sein. Auch kann die Oberfläche einer Holzwerkstoff-Trägerplatte geschliffen sein (ohne Presshaut) oder auch nicht-geschliffen sein (mit Presshaut). Im Falle einer Kunststoff-Trägerplatte kann die Oberfläche koronabehandelt sein.

### Poröses Beschichtungsmaterial

Das mindestens eine poröse Beschichtungsmaterial kann ausgewählt sein aus den folgenden Materialien: eine Furnierlage, ein Ledermaterial, ein Papiermaterial, wie Kartonage, Filzmaterial, Vliesmaterial und andere Stoffmaterialien. Insbesondere sind Materialien umfasst, die eine Porosität aufweisen, in der flüssiger Klebstoff während des Verpressens aufsteigen kann und die zumindest teilweise plastisch verformbar sind.

Im Falle der Verwendung einer Furnierlage umfasst diese in einer Ausführungsform mindestens eine Lage aus Echtholzfurnier.

In einer weitergehenden Ausführungsform umfasst das mindestens eine Furnier mindestens eine Echtholz-Lage mit einer Dicke zwischen 0,2-10 mm, bevorzugt 0,5-5 mm, insbesondere bevorzugt 0,5-2 mm. Das Furnier kann einstückig von einem Stamm, zum Beispiel durch Schälen hergestellt sein. Es kann aber auch aus einzelnen Stücken zusammengesetzt sein, die zum Beispiel durch Bindemittel oder sogenannte Leimfäden miteinander verbunden sind. Das Furnier weist bevorzugt die Abmessungen der Trägerplatte auf. Das Furnier weist eine der Trägerplatte zugewandte Unterseite und eine der Trägerplatte abgewandte Oberseite auf.

Im Falle der Verwendung vom Ledermaterialien, z.B. als Dämmschicht, wird bevorzugt ein Lederfaserwerkstoff mit einer Dicke zwischen 0,5 mm und 1 mm, bevorzugt 0,75 mm verwendet.

Als Lederwerkstoff oder auch Lederfaserstoff wird hierbei ein Werkstoff aus Falzspänen z.B. Chromfalzspänen und zerkleinerten, pflanzlich gegerbten Lederresten der lederverarbeitenden Industrie, Bindemittel z.B. Naturlatex und natürlichen Fetten definiert. Der Anteil an Leder in einem Lederfaserstoff beträgt mindestens 50%. Die verarbeiteten Lederreste können unter anderem vom Rind oder auch anderen Tieren, wie zum Beispiel Pferden, stammen. Es können aber auch Naturleder in unterschiedlichen Dicken verwendet werden.

In einer weitergehenden Ausführungsform des vorliegenden Verfahrens werden die mindestens eine Trägerplatte, die mindestens eine auf der Trägerplatte angeordnete Klebstoffschicht und das mindestens eine poröse Beschichtungsmaterial bei Temperaturen zwischen 150 und 200°C, bevorzugt zwischen 170 und 180°C bei einem Druck von 30 bis 50 kg/cm², bevorzugt 40 kg/cm² für 30-120 Sekunden, bevorzugt 60 bis 90 Sekunden verpresst.

Das vorliegende Verfahren ermöglicht somit die Bestimmung des Penetrationsgrades von Klebstoff in ein mit einer Trägerplatte verpresstem porösen Beschichtungsmaterials mit folgenden Schichtaufbau: Holzwerkstoffplatte - Klebstoffsystem - ggfs. Additive - poröses Beschichtungsmaterial.

Die NIR-Messung der Penetrationshöhe des Klebstoffes in das poröse Beschichtungsmaterial kann in einer Variante kontinuierlich innerhalb, d.h. online, der Produktionslinie der Werkstoffplatten bestimmt werden. In dieser online-Variante wird demnach die Penetrationshöhe im laufenden Produktionsprozess bestimmt. Dies ermöglicht eine direkte Steuerung und Eingriff in den Produktionsprozess.

In einer zweiten Ausführungsvariante des vorliegenden Verfahrens kann die Penetrationshöhe auch außerhalb (d.h. offline) der Produktionslinie der Werkstoffplatten bestimmt werden. In dieser Variante wird demnach eine fertig verpresste Werkstoffplatte aus der Produktionslinie entnommen bzw. ausgeschleust und offline z.B. in einem separaten Labor im Rahmen einer routinemäßigen Qualitätskontrolle vermessen.

In einer weiteren Variante kann die NIR-Messung sowohl online als auch offline erfolgen.

Es kann auch vorgesehen sein, dass der mindestens eine NIR-Messkopf sich quer zur Laufrichtung der mit dem porösen Beschichtungsmaterial verpressten Trägerplatten bewegt. Der NIR-Detektor kann an beliebigen Stellen in Transportrichtung der Platte installiert werden. Dabei kann der Detektor auch über die Plattenbreite traversieren bzw. bestimmte Problembereiche analysieren (z. B. im Rand- oder Mittelbereich der Platten usw.). Außerdem stehen die Messwerte sofort zur Verfügung und erlauben einen sofortigen Eingriff in den Prozess. Dies ist bei anderen Verfahren nicht ohne weiteres möglich.

Das vorliegende Verfahren wird in einer Produktionslinie umfassend mindestens einen NIR-Multimesskopf, bevorzugt mindestens zwei NIR-Multimessköpfe, und mindestens ein Steuerungssystem durchgeführt. Eine solche Produktionslinie kann eine Produktionslinie zur Herstellung von Werkstoffplatten sein. Bevorzugt wird das vorliegende Verfahren zur Bestimmung der Klebstoffpenetration in das poröse Beschichtungsmaterial kontinuierlich und online durchgeführt.

Das Steuerungssystem der Produktionslinie umfasst mindestens eine computergestützte Auswerteeinheit (bzw. Prozessoreinheit) und eine Datenbank. In der Auswerteeinheit erfolgt der Abgleich bzw. Vergleich des für das Produkt (d.h. verpresstes poröses Beschichtungsmaterial) gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten können zur Steuerung der Produktionslinie verwendet werden. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der Produktionslinie wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der Produktionslinie gespeichert.

Die Erfindung wird nachfolgend unter Bezugnahme auf die Figuren der Zeichnungen an einem Ausführungsbeispiel näher erläutert. Es zeigt:

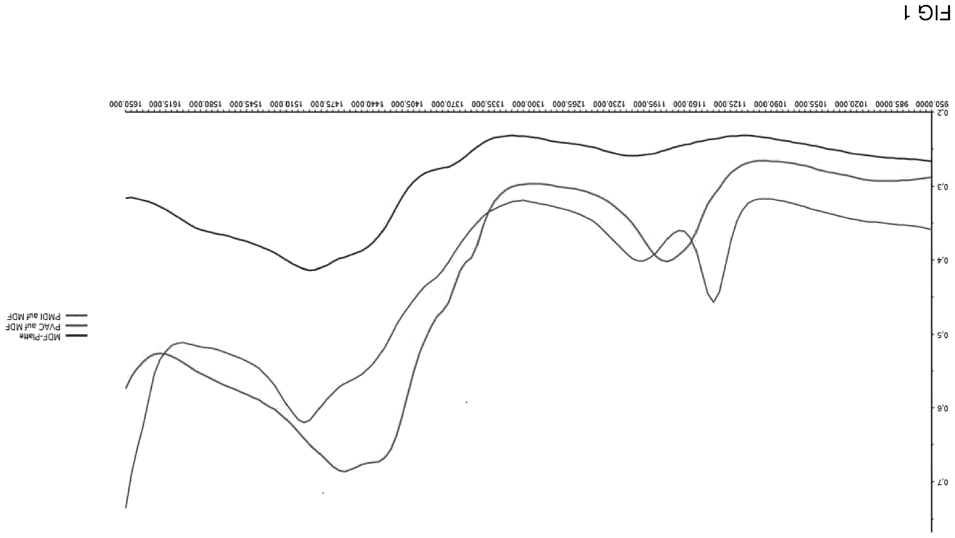

- Figur 1: NIR-Spektren einer MDF-Platte, einer mit einem Polyurethan-Klebstoff (PMDI) versehenen MDF-Platte und einer mit einem Polyvinylacetat-Klebstoff versehenen MDF-Platte.

Figur 1 zeigt NIR-Spektren einer MDF-Platte ohne Klebstoff, einer mit einem Polyurethan-Klebstoff (PMDI) versehenen MDF-Platte und einer mit einem Polyvinylacetat-Klebstoff versehenen MDF-Platte.

Im Falle des PMDI-Klebstoffs sind im NIR-Spektrum charakteristische Peaks in Wellenlängenbereichen zwischen 1120 und 1250 nm mit Maxima bei 1130-1150 nm und 1200-1220 nm, und zwischen 1440 und 1540 nm zu erkennen.

Im Falle des PVAc - Klebstoffs sind im NIR-Spektrum charakteristische Peaks in Wellenlängenbereichen zwischen 1160 und 1220 nm mit einem Maximum bei 1180-1200 nm, und zwischen 1420 und 1480 nm zu erkennen.

### Ausführungsbeispiel 1:

Auf zwei 8 mm HDF (500 × 500 mm) wurde einseitig schwarz eingefärbtes PMDI (100% System) in Mengen von 50 und 100 fl g/m² aufgebracht. Dies erfolgte mit einem Walzenauftragsaggregat.

Auf die HDF's wurde dann ein Eichenfurnier (Stärke: 0,5 mm) aufgelegt. Dann wurde der Aufbau in einer Laborpresse bei 180°C, einem Druck von 40 kg/cm² und einer Presszeit von 160 Sekunden verpresst. Das Furnier wurde dadurch auf eine Stärke von 0,35 mm komprimiert.

Danach wurden aus den Platten Proben geschnitten (100 x100 mm, je vier Stück). Nach dem Abkühlen wurde die Oberfläche mit einem NIR-Messkopf an vier Stellen vermessen, die durch ein Koordinatenkreuz gekennzeichnet waren und an denen später der Abrieb/Abtrag durch den Taber-Abraser erfolgt. Zusätzlich wurde eine Nullprobe mit vermessen bei der das Eichenfurnier mit Hilfe einer Klebvorrichtung auf der HDF fixiert war.

Anschließend wurden die Proben auf einem Taber-Abraser geprüft. Die Prüfung erfolgte in Anlehnung an die DIN EN 13329. Die Reibräder des Taber-Abrasers wurden mit den üblichen Schleifpapieren beklebt und auch mit den üblichen Gewichten belastet. Dann wurde jeweils nach 200 Umdrehungen visuell geprüft, ob bereits schwarze Verfärbungen im Furnier zu beobachten waren. Dann wurde mit einer Messuhr in der durch das Schleifpapier erzeugten kreisförmigen Vertiefung in den vier Kreissegmenten, die durch ein Koordinatenkreuz gebildet werden, der Abtrag in mm bestimmt und daraus der Mittelwert gebildet. Aus diesem Mittelwert wurde zusammen mit den vier anderen Proben ein Gesamtmittelwert gebildet. Von der Furnierstärke, die mit Hilfe eines Mikroskops bestimmt wurde, wurde dann der Abtrag subtrahiert und dann mit den Spektren korreliert.

Die ermittelten Werte sind in der folgenden Tabelle 1 zusammengefasst. Es ist ersichtlich, dass bei höheren Mengen (100 g/m²) von aufgetragenen PMDI-Klebstoff die mechanische Abnahme in Taber-Abraser Test geringer ausfällt, als bei 50 g/m² an PMDI-Klebstoff. Dies belegt, dass je mehr PMDI-Klebstoff aufgetragen wird, desto mehr PMDI-Klebstoff dringt in die Furnierlage ein und desto weniger muss im Taber-Abraser Test abgetragen werden, um die schwarzen Verfärbungen in der Furnierlage zu beobachten.

Die mechanische Abnahme in Taber-Abraser Test korrespondiert dabei mit der durch das NIR-Verfahren ermittelten Abnahme, so dass das NIR-Verfahren einen Nachweis der Penetrationshöhe des Klebstoffes in der Furnierlage zulässt.

**Tabelle 1**

| Menge an aufgetragenem PMDI-Klebstoff | Abnahme in mm NIR | Abnahme in mm Taber-Abraser | Differenz in mm |
|---|---|---|---|
| Nullprobe | 0,26 | 0,29 | 0,03 |
| 50 g PMDI fl/m² | 0,11 | 0,14 | 0,03 |
| 100 g PMD fl/m² | 0,06 | 0,06 | 0 |

Der Messkopf zur Bestimmung der Klebstoffpenetration wird unmittelbar hinter der verwendeten Presse installiert. Durch eine automatisierte Verschiebemöglichkeit kann der Messkopf unterschiedliche Bereiche eine mit Furnier beschichteten Platte analysieren oder er kann über die Platte traversieren. Dadurch ist sichergestellt, dass auch Bereiche, die üblicherweise wegen unterschiedlichen Pressbedingungen problematisch sein können (z. B. Plattenränder) analysiert werden.

Bei einer nicht adäquaten Penetration des Klebstoffes in das Furnier kann durch Veränderung der Presstemperatur und/oder der Presszeit ein verbesserter Klebstofffluss erreicht werden. Dabei werden die beiden Parameter gegenläufig verändert. Bei einer Reduktion der Presstemperatur wird die Presszeit verlängert. Beispielsweise wird bei einer Reduzierung der Presstemperatur um 10°C die Presszeit um 10 bis 20 Sekunden verlängert.

### Ausführungsbeispiel 2:

Zur Prüfung der Genauigkeit der Kalibration wurde statt einer Beschichtung eines HWS mit einem Furnier eine Beschichtung mit einem Leder durchgeführt. Dabei wurde zunächst mit Hilfe eines NIR-Messgerätes ein Spektrum des eingesetzten Leders erstellt, um zu prüfen, ob der PMDI-Peak bei ca. 1500 nm durch Peaks des Leders überlagert wird. Was sich nicht bestätigt hat.

Auf eine 8 mm HDF (500 × 500 mm) wurde einseitig ein PMDI-Auftrag von 100 g fl/m² (Feststoffgehalt 100%).

Auf das PMDI wurde dann ein braunes Leder (Stärke: 0,75 mm) aufgelegt. Dann wurde der Aufbau in einer Laborpresse bei 180°C, einem Druck von 40 kg/cm² und einer Presszeit von 160 Sekunden verpresst. Das Leder wurde dadurch auf eine Stärke von 0,45 mm komprimiert.

Danach wurden aus der Platte Proben geschnitten (100 x100 mm, je vier Stück). Nach dem Abkühlen wurde die Oberfläche mit einem NIR-Messkopf an vier Stellen vermessen, die durch ein Koordinatenkreuz gekennzeichnet waren.

Die Messung mit dem NIR-Messgerät ergab eine Eindringtiefe von 0,35 mm. Dies wurde anschliessend mit dem Taber-Abraser überprüft. Dabei wurde ein Wert von 0,35 ermittelt.

Andere poröse Beschichtungsmaterialien wie Stoff, Filz, Vlies usw. können ebenfalls über dieses Verfahren vermessen werden.

## Patentansprüche

1. Verfahren zur Bestimmung der Klebstoffpenetration in Form der Penetrationshöhe des Klebstoffes in mindestens ein poröses Beschichtungsmaterial, welches mit mindestens einer Trägerplatte und mindestens einer auf der Trägerplatte angeordneten Klebstoffschicht verpresst ist, wobei während des Pressvorganges der Klebstoff in das mindestens eine poröse Beschichtungsmaterial penetriert bzw. aufsteigt,
umfassend die Schritte
- Aufnahme von mindestens einem NIR-Spektrum von mehreren Referenzproben mit jeweils unterschiedlichem Werten für die Klebstoffpenetration in ein poröses Beschichtungsmaterial unter Verwendung von mindestens einem NIR-Messkopf in einem Wellenlängenbereich zwischen 500 nm und 2500 nm, bevorzugt zwischen 700 nm und 2000 nm, insbesondere bevorzugt zwischen 900 nm und 1700 nm, und besonders vorteilhaft zwischen 1000 nm und 1300 nm und/oder zwischen 1400 nm und 1550 nm; wobei die Referenzproben gleichartig sind zu der zu vermessenden Probe;
- Bestimmung der Klebstoffpenetration in das poröse Beschichtungsmaterial der genannten Referenzproben mittels eines mechanischen Abtrages der porösen Materialoberfläche;
- Zuordnung der mittels mechanischen Abtrages bestimmten Klebstoffpenetration zu den aufgenommenen NIR-Spektren der genannten Referenzproben; und
- Erstellung eines Kalibriermodells für den Zusammenhang zwischen den spektralen Daten der NIR-Spektren und den dazugehörigen Klebstoffpenetrationen der Referenzproben mittels einer multivariaten Datenanalyse;
- Verpressen von mindestens einem porösen Beschichtungsmaterial mit mindestens einer Trägerplatte und mindestens einer auf der Trägerplatte angeordneten Klebstoffschicht,
- Aufnehmen von mindestens einem NIR-Spektrum des mit der Trägerplatte und der Klebstoffschicht verpressten porösen Beschichtungsmaterials unter Verwendung des mindestens einen NIR-Messkopfes in einem Wellenlängenbereich zwischen 500 nm und 2500 nm, bevorzugt zwischen 700 nm und 2000 nm, insbesondere bevorzugt zwischen 900 nm und 1700 nm und besonders vorteilhaft zwischen 1000 nm und 1300 nm und/oder zwischen 1400 nm und 1550 nm; und
- Bestimmen der Klebstoffpenetration in das mindestens eine poröse Beschichtungsmaterial durch Vergleich des für das poröse Beschichtungsmaterialaufgenommenen NIR-Spektrums mit dem erstellten Kalibriermodell.

2. Verfahren nach Anspruch 1, **dadurch gekennzeichnet, dass** das NIR-Spektrum in einem Wellenlängenbereich zwischen 1100 und 1250 nm und 1400 und 1550 nm aufgenommen wird.

3. Verfahren nach einem der vorhergehenden Ansprüche, **dadurch gekennzeichnet, dass** der mindestens eine Klebstoff ein Polyurethan-Klebstoff ist.

4. Verfahren nach Anspruch 3, **dadurch gekennzeichnet, dass** der der Polyurethan-Klebstoff auf der Basis von aromatischen Polyisocyanaten, insbesondere Polydiphenylmethandiisocyanat (PMDI), Toluylendiisocyanat (TDI) und/oder Diphenylmethandiisocyanat (MDI) vorliegt, wobei PMDI besonders bevorzugt ist.

5. Verfahren nach einem der Ansprüche 1-2, **dadurch gekennzeichnet, dass** der Klebstoff ein Polyvinylester-Klebstoff, insbesondere ein Polyvinylacetat-Klebstoff ist.

6. Verfahren nach einem der vorhergehenden Ansprüche, **dadurch gekennzeichnet, dass** auf die mindestens eine Klebstoffschicht mindestens ein Additiv aufgebracht wird.

7. Verfahren nach Anspruch 6, **dadurch gekennzeichnet, dass** das mindestens eine Additiv ausgewählt ist aus folgender Gruppe umfassend Farbstoffe (z.B.Tinte), Pigmente (z.B. Farbpigmente, Metallpigmente oder reflektierende Pigmente) Flammschutzmittel (z.B. Ammoniumpolyphosphat, Tris(tri-bromneopentyl)phosphat, Zinkborat oder Borsäurekomplexe von mehrwertigen Alkoholen), Mittel zur Erhöhung der Leitfähigkeit, UV-Stabilisatoren, Bleichmittel, Hydrophobierungsmittel oder antimikrobielle Wirkstoffe.

8. Verfahren nach Anspruch 6-7, **dadurch gekennzeichnet, dass** das mindestens eine Additiv ein Farbstoff ist.

9. Verfahren nach einem der vorhergehenden Ansprüche, **dadurch gekennzeichnet, dass** die mindestens eine Trägerplatte eine Platte aus einem Holzwerkstoff, insbesondere eine Span-, mitteldichte Faser (MDF)-, hochdichte Faser (HDF)-, (OSB- oder Sperrholzplatte, aus Kunststoff, einem Holzwerkstoff-Kunststoff-Gemisch oder einem Verbundwerkstoff, , eine Zementfaserplatte, Gipsfaserplatte oder eine WPC-Platte (Wood Plastic Composites) oder eine SPC-Platte (Stone Plastic Composites) ist.

10. Verfahren nach einem der vorhergehenden Ansprüche, **dadurch gekennzeichnet, dass** das mindestens eine poröse Beschichtungsmaterial mindestens eine Furnierlage, ein Ledermaterial, Filzmaterial, Vliesmaterial und/oder solche Materialien umfasst, die eine Porosität aufweisen, in der flüssiger Klebstoff während des Verpressens aufsteigen kann und die zumindest teilweise plastisch verformbar sind.

11. Verfahren nach einem der vorhergehenden Ansprüche, **dadurch gekennzeichnet, dass** die mindestens eine Trägerplatte, die mindestens eine auf der Trägerplatte angeordnete Klebstoffschicht und das mindestens eine poröse Beschichtungsmaterial bei Temperaturen zwischen 150 und 200°C, bevorzugt zwischen 170 und 180°C bei einem Druck von 30 bis 50 kg/cm², bevorzugt 40 kg/cm² für 30-120 Sekunden, bevorzugt 60 bis 90 Sekunden verpresst werden.

12. Verfahren nach einem der vorhergehenden Ansprüche, **dadurch gekennzeichnet, dass** für die Erstellung des Kalibriermodells spektrale Daten aus dem gesamten aufgenommenen Spektralbereich verwendet werden.

13. Verfahren nach einem der vorhergehenden Ansprüche, **dadurch gekennzeichnet, dass** für die Erstellung des Kalibriermodells spektrale Daten aus dem NIR-Spektralbereich zwischen 1000 nm und 1300 nm und/oder zwischen 1400 nm und 1550 nm verwendet werden, die mittels geeigneter mathematischer Methoden vorbehandelt werden und anschließend der multivariaten Datenanalyse zugeführt werden.

14. Verfahren nach einem der vorhergehenden Ansprüche, **dadurch gekennzeichnet, dass** die Bestimmung der Klebstoffpenetration in das poröse Beschichtungsmateriale kontinuierlich und online erfolgt.

## Claims

1. Method for determining the adhesive penetration in the form of the penetration height of the adhesive into at least one porous coating material which is pressed with at least one carrier plate and at least one adhesive layer arranged on the carrier plate, wherein the adhesive penetrates or rises into the at least one porous coating material during the pressing process,
comprising the steps
- Recording at least one NIR spectrum of several reference samples, each having different values for adhesive penetration into a porous coating material, using at least one NIR measuring head in a wavelength range between 500 nm and 2500 nm, preferably between 700 nm and 2000 nm, particularly preferably between 900 nm and 1700 nm, and especially advantageously between 1000 nm and 1300 nm and/or between 1400 nm and 1550 nm; the reference samples being identical to the sample to be measured;
- Determining of adhesive penetration into the porous coating material of said reference samples by means of a mechanical removal of the porous material surface;
- Correlating of the adhesive penetration determined by mechanical removal with the recorded NIR spectra of said reference samples; and
- Establishing of a calibration model for the relationship between the spectral data of the NIR spectra and the corresponding adhesive penetrations of the reference samples using multivariate data analysis;
- Pressing of at least one porous coating material with at least one carrier plate and at least one adhesive layer arranged on the carrier plate,
- Recording at least one NIR spectrum of the porous coating material pressed with the carrier plate and the adhesive layer using the at least one NIR measuring head in a wavelength range between 500 nm and 2500 nm, preferably between 700 nm and 2000 nm, in particular preferably between 900 nm and 1700 nm and especially advantageously between 1000 nm and 1300 nm and/or between 1400 nm and 1550 nm; and
- Determining the adhesive penetration into the at least one porous coating material by comparing the NIR spectrum recorded for the porous coating material with the calibration model created.

2. Method according to claim 1, **characterized in that** the NIR spectrum is recorded in a wavelength range between 1100 and 1250 nm and 1400 and 1550 nm.

3. Method according to one of the preceding claims, **characterized in that** the at least one adhesive is a polyurethane adhesive.

4. Method according to claim 3, **characterized in that** the polyurethane adhesive is based on aromatic polyisocyanates, in particular polydiphenylmethane diisocyanate (PMDI), toluylene diisocyanate (TDI) and/or diphenylmethane diisocyanate (MDI), PMDI being particularly preferred.

5. Method according to one of claims 1-2, **characterized in that** the adhesive is a polyvinyl ester adhesive, in particular a polyvinyl acetate adhesive.

6. Method according to one of the preceding claims, **characterized in that** at least one additive is applied to the at least one adhesive layer.

7. Method according to claim 6, **characterized in that** the at least one additive is selected from the following group comprising dyes (e.g.ink), pigments (e.g. color pigments, metallic pigments or reflective pigments) flame retardants (e.g. ammonium polyphosphate, tris(tri-bromo neopentyl) phosphate, zinc borate or boric acid complexes of polyhydric alcohols), agents for increasing conductivity, UV stabilizers, bleaching agents, hydrophobing agents or antimicrobial agents.

8. Method according to claim 6-7, **characterized in that** the at least one additive is a dye.

9. Method according to one of the preceding claims, **characterized in that** the at least one carrier plate is a plate made of a wood material, in particular a particleboard, medium-density fiberboard (MDF), high-density fiberboard (HDF), (OSB) or plywood board, made of plastic, a wood material-plastic mixture or a composite material, a cement fiberboard, gypsum fiberboard or a WPC board (Wood Plastic Composites) or an SPC board (Stone Plastic Composites).

10. Method according to one of the preceding claims, **characterized in that** the at least one porous coating material comprises at least one veneer layer, leather material, felt material, nonwoven material and/or such materials which have a porosity in which liquid adhesive can rise during pressing and which are at least partially plastically deformable.

11. Method according to one of the preceding claims, **characterized in that** the at least one carrier plate, the at least one adhesive layer disposed on the carrier plate, and the at least one porous coating material are compressed at temperatures between 150 and 200°C, preferably between 170 and 180°C, at a pressure of 30 to 50 kg/cm² , preferably 40 kg/cm² for 30-120 seconds, preferably 60 to 90 seconds.

12. Method according to one of the preceding claims, **characterized in that** spectral data from the entire recorded spectral range are used to create the calibration model.

13. Method according to one of the preceding claims, **characterized in that** spectral data from the NIR spectral range between 1000 nm and 1300 nm and/or between 1400 nm and 1550 nm, which are pretreated by means of suitable mathematical methods and are subsequently fed to the multivariate data analysis, are used to create the calibration model.

14. Method according to one of the preceding claims, **characterized in that** the determination of the adhesive penetration into the porous coating material is performed continuously and online.

## Revendications

1. Procédé de détermination de la pénétration de colle sous forme de hauteur de pénétration de la colle dans au moins un matériau de revêtement poreux, lequel est lié par pression à au moins un panneau de support et au moins à une couche de colle disposée sur le panneau de support, dans lequel la colle pénètre ou remonte dans l'au moins un matériau de revêtement poreux pendant le processus de liaison par pression,
comprenant les étapes
- d'enregistrement d'au moins un spectre infrarouge proche de plusieurs échantillons de référence avec respectivement une valeur différente pour la pénétration de colle dans un matériau de revêtement poreux en utilisant au moins une tête de mesure infrarouge proche dans une plage de longueurs d'onde entre 500 nm et 2500 nm, de manière préférée entre 700 nm et 2000 nm, en particulier de manière préférée entre 900 nm et 1700 nm, et de manière particulièrement préférée entre 1000 nm et 1300 nm et/ou entre 1400 nm et 1550 nm ; dans lequel les échantillons de référence sont similaires par rapport à l'échantillon à mesurer ;
- de détermination de la pénétration de colle dans le matériau de revêtement poreux desdits échantillons de référence au moyen d'un enlèvement mécanique de la surface de matériau poreuse ;
- d'attribution de la pénétration de colle déterminée au moyen de l'enlèvement mécanique aux spectres infrarouges proches enregistrés desdits échantillons de référence ; et
- de création d'un modèle d'étalonnage pour la corrélation entre les données spectrales des spectres infrarouges proches et les pénétrations de colle correspondantes des échantillons de référence au moyen d'une analyse de données à variables multiples ;
- de liaison par pression d'au moins un matériau de revêtement poreux à au moins un panneau de support et à au moins une couche de colle disposée sur le panneau de support ;
- d'enregistrement d'au moins un spectre infrarouge proche du matériau de revêtement poreux lié par pression au panneau de support et à la couche de colle en utilisant l'au moins une tête de mesure infrarouge proche dans une plage de longueurs d'onde entre 500 nm et 2500 nm, de manière préférée entre 700 nm et 2000 nm, en particulier de manière préférée entre 900 nm et 1700 nm et de manière particulièrement avantageuse entre 1000 nm et 1300 nm et/ou entre 1400 nm et 1550 nm ; et
- de détermination de la pénétration de colle dans l'au moins un matériau de revêtement poreux en comparant le spectre infrarouge proche enregistré pour le matériau de revêtement poreux au modèle d'étalonnage créé.

2. Procédé selon la revendication 1, **caractérisé en ce que** le spectre infrarouge proche est enregistré dans une plage de longueurs d'onde entre 1100 et 1250 nm et 1400 et 1550 nm.

3. Procédé selon l'une quelconque des revendications précédentes, **caractérisé en ce que** l'au moins une colle est une colle à base de polyuréthane.

4. Procédé selon la revendication 3, **caractérisé en ce que** la colle à base de polyuréthane présente est à base de polyisocyanates aromatiques, en particulier de diisocyanate de polydiphénylméthane (PMDI), de diisocyanate de toluène (TDI) et/ou de diisocyanate de diphénylméthane (MDI), dans lequel le PMDI est en particulier préféré.

5. Procédé selon l'une quelconque des revendications 1 ou 2, **caractérisé en ce que** la colle est une colle de polyester de vinyle, en particulier une colle à base de polyacétate de vinyle.

6. Procédé selon l'une quelconque des revendications précédentes, **caractérisé en ce qu'**au moins un additif est appliqué sur l'au moins une couche de colle.

7. Procédé selon la revendication 6, **caractérisé en ce que** l'au moins un additif est choisi parmi un groupe suivant comprenant des colorants (par exemple de l'encre), des pigments (par exemple des pigments de couleur, des pigments métalliques ou des pigments réfléchissants), des retardateurs de flammes (par exemple du polyphosphate d'ammonium, du tris(tri-bromnéopentyle)phosphate, du borate de zinc ou des complexes d'acide borique d'alcools polyvalents), des agents pour augmenter la conductivité, des agents stabilisants UV, des agents de blanchiment, des agents rendant hydrophobe ou des principes actifs antimicrobiens.

8. Procédé selon la revendication 6 ou 7, **caractérisé en ce que** l'au moins un additif est un colorant.

9. Procédé selon l'une quelconque des revendications précédentes, **caractérisé en ce que** l'au moins un panneau de support est un panneau composé d'un matériau dérivé du bois, en particulier un panneau d'aggloméré, un panneau de fibres à densité moyenne (MDF), un panneau de fibres à haute densité (HDF), un panneau à lamelles de bois orientées (panneau OSB) ou un panneau de contreplaqué, un panneau composé de matière plastique, d'un mélange de matériau dérivé du bois et de matière plastique ou d'un matériau composite, un panneau en fibres de ciment, un panneau en fibres de plâtre ou un panneau en matériau composite plastique/bois stratifié (WPC) ou un panneau en matériau composite pierre/plastique (SPC).

10. Procédé selon l'une quelconque des revendications précédentes, **caractérisé en ce que** l'au moins un matériau de revêtement poreux comprend au moins une couche de placage, un matériau en cuir, un matériau en feutre, en matériau en non-tissé et/ou des matériaux tels qu'ils présentent une porosité, dans laquelle de la colle liquide peut remonter au cours de la liaison par pression et qui peut être déformée plastiquement au moins en partie.

11. Procédé selon l'une quelconque des revendications précédentes, **caractérisé en ce que** l'au moins un panneau de support, l'au moins une couche de colle disposée sur le panneau de support et l'au moins un matériau de revêtement poreux sont liés par pression à des températures entre 150 et 200 °C, de manière préférée entre 170 et 180 °C à une pression de 30 à 50 kg/cm², de manière préférée de 40 kg/cm² pendant 30-120 secondes, de manière préférée de 60 à 90 secondes.

12. Procédé selon l'une quelconque des revendications précédentes, **caractérisé en ce que** des données spectrales issues de la totalité du domaine spectral enregistré sont utilisées pour la création du modèle d'étalonnage.

13. Procédé selon l'une quelconque des revendications précédentes, **caractérisé en ce que** des données spectrales issues du domaine spectral infrarouge proche entre 1000 nm et 1300 nm et/ou entre 1400 nm et 1550 nm sont utilisées pour la création du modèle d'étalonnage, lesquelles sont prétraitées au moyen de méthodes mathématiques adaptées et sont amenées par la suite à une analyse de données à variables multiples.

14. Procédé selon l'une quelconque des revendications précédentes, **caractérisé en ce que** la détermination de la pénétration de colle dans le matériau de revêtement poreux est effectuée en continu ou en ligne.
